(19) **Europäisches Patentamt European Patent Office Office européen des brevets**

(11) **EP 4 276 444 B1**

(12) **EUROPEAN PATENT SPECIFICATION**

(45) Date of publication and mention of the grant of the patent:
**25.02.2026 Bulletin 2026/09**

(51) International Patent Classification (IPC):
**G01N 21/3504** (2014.01)   **G01N 33/00** (2006.01)
**G01N 21/31** (2006.01)

(21) Application number: **23169998.4**

(52) Cooperative Patent Classification (CPC):
**G01N 21/3504; G01N 33/004;** G01N 2021/3166

(22) Date of filing: **26.04.2023**

(54) **OPTICAL CO2 CONCENTRATION METER BASED ON IR LIGHT ABSORPTION IN GAS**

OPTISCHER CO2-KONZENTRATIONSMESSER AUF DER BASIS VON IR-LICHTABSORPTION IN GAS

DISPOSITIF DE MESURE DE CONCENTRATION DE CO2 OPTIQUE BASÉ SUR L'ABSORPTION DE LUMIÈRE INFRAROUGE DANS UN GAZ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **13.05.2022 LT 2022520**

(43) Date of publication of application:
**15.11.2023 Bulletin 2023/46**

(73) Proprietor: **Valstybinis Moksliniu Tyrimu Institutas Fiziniu Ir Technologijos Mokslu Centras 02300 Vilnius (LT)**

(72) Inventors:
• **REMEIKIS, Vidmantas**
  **02300 Vilnius (LT)**
• **PLUKIS, Arturas**
  **02300 Vilnius (LT)**
• **PLUKIENE, Rita**
  **02300 Vilnius (LT)**
• **URBA, Andriejus**
  **02300 Vilnius (LT)**

(74) Representative: **Draugeliene, Virgina Adolfina Tarpine Ltd A. P. Kavoliuko g. 24-152 04328 Vilnius (LT)**

(56) References cited:
KR-B1- 101 835 556     US-A- 5 650 624
US-A- 5 721 430         US-A- 5 747 809
US-A1- 2003 205 673     US-A1- 2010 188 232

**Description**

Technical field to which invention relates

The invention relates to optically determined gas concentration measuring devices and is intended for the estimation of $CO_2$ concentration in a particularly wide range of concentrations. Invention refers to devices that are able to measure the concentrations of optically detectable gases and measurements can be used in pollutant/waste characterization. The optical $CO_2$ concentration meter can be used as independent device for room air control or also as an auxiliary - $CO_2$ concentration measurement module, which could be an integrated part of other devices e.g. mass spectrometers, total carbon analyzers or the specific activity of radiocarbon analyzers, which measures high $CO_2$ gas concentration ($<10^3$ppm) coming from sample oxidation cameras.

The invention will be particularly useful for industrial and scientific applications where quick determination in the compact setup and in a small sample volume is needed and the expected concentration of analyte is not known beforehand and can be variable in a wide range. The device would be particularly useful as a $CO_2$ concentration detection module for the system described in our invention LT2022 519.

Indication of the background art

Optical gas absorption detectors determine the concentration of a gaseous analyte in the gas sample by measuring the attenuation of the light passing through the gas sample due to light absorption by analyte of interest. Many of such detectors intended for molecular gases use light sources in the infrared region of 0.8 to 10 $\mu$m, in which range the gases such as carbon dioxide, carbon monoxide, nitrogen oxides, methane and others feature vibrational - rotational IR absorption bands.

However, the optic absorption method itself (in contrast to some other methods like fluorescence) inherently features not very wide dynamic detection range. The concentration of the analyzed gas is determined according to the Beer-Lambert-Bouguer law which is exponential:

$$I=I_0 exp(-\alpha lc) \qquad (1)$$

here $I$ is the light intensity transmitted through the gas cell, $I_0$ is the light incident on the gas cell $l$ - the length of the light, c is the analyte concentration in the gas, and $\alpha$ is the absorption coefficient of the sample (units of cm$^{-1}$). At low values of $\alpha l$, equation (1) can be approximated to a linear relationship:

$$I=I_0(1-\alpha lc) \qquad (2)$$

In practice, this means that in case one makes a short enough (thin) optic cell with small $l$, then one for sure gets linear relationship with relatively high concentrations, but low concentrations of gaseous analyte may occur below the detection limit, because they would induce too small attenuation of light to be detected by optic electronic system. In contrast, if one makes a long optic cell, then high concentrations become impossible to detect precisely, because they absorb big part of light, running into unlinearity range of Beer-Lambert-Bouguer formula and generating large analytical errors.

The problem can be solved in different ways. For example, a combination of different length optic cells can be used. This complicates the design by the need to use a sophisticated gas flow transfer system among the cells and building many cells and multiple optic elements, typically each different length optic cell will require some elements on its own. Moreover, if one builds two-beam system (comprising "sample" and "reference" optic cells) then the total number of optic cells and optic elements doubles.

Another possible solution can be dilution of samples which in turn requires special preparations, introduces potential challenges and errors by itself.

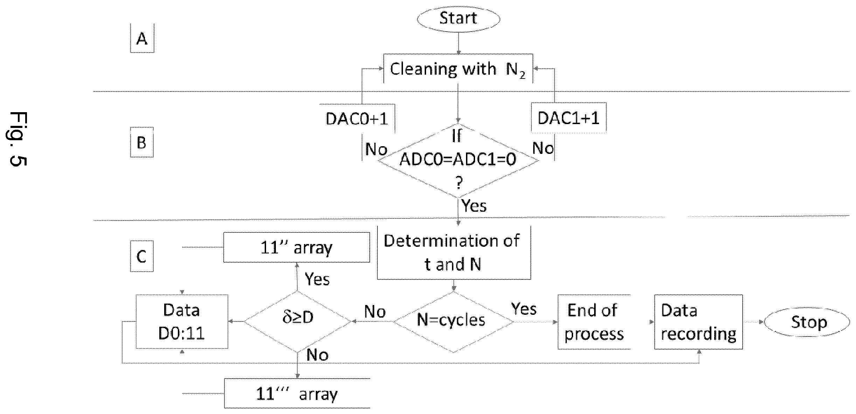

There is known an apparatus and method for measuring isotopic ratios in gaseous samples (US Patent No. US 5 747 809 A, 05-05-1998, SRI International). It is a system with four optic cells of different lengths, a mechanical (rotating) optical chopper and pneumatic valves. The invention is aimed at the optical determining of isotopical ratios of different molecular gaseous species. In particular, for determining of $^{12}CO_2/$ $^{13}CO_2$ isotopical ratio, it uses IR absorption region 4.1-4.3 $\mu$m for $^{12}CO_2$ and 4.23-4.43 $\mu$m absorption region for $^{13}CO_2$. The above invention however focuses on determining of the ratio of different isotopic species concentration rather than determining the absolute value of $CO_2$ concentration. The suggested device is working in a relatively narrow range of analyte concentrations (about one order of magnitude, see their Figure 5 A, B) and is not intended for increasing the range of measured concentrations in any way.

There is also known a respiratory gas analyzer, see. patent application No.US 2003/205673 A1, 06-11-2003, by Kevin G. Williams. The proposed device is an IR absorption photometer with a system of multiple dichroic mirrors and multiple detectors aimed at the measurement of numerous species of gases in the exhaled air. The invention deals with the drift of optic system with thermopile detectors and attempts to offer a costefficient solution. For the $CO_2$ detection, IR band of $CO_2$ referred to as 4.3 $\mu$m is employed. No attempts are seen to extend the range of $CO_2$ concentration measurements and no any different bands or any different isotopic species of $CO_2$ are considered.

Korean patent No. KR 101 835 556 B1, 28-02-2018, describes a non-dispersive infrared multi gas detector having one light path. It uses a sophisticated system of parabolic mirrors and elaborated calculations to determine a multitude of ambient gas concentrations ($CH_4$, CO, $CO_2$ and other). In particular, for the detection of $CO_2$, the invention employs absorption band of $CO_2$ referred to as 4.23 $\mu$m and they do not employ any other spectral band of $CO_2$ and do not discuss any different isotopic species of $CO_2$. No attempts are seen to extend the measurement range into higher concentrations.

The nearest known system and method according to the technical field is described in patent application No.US 2010/188232 A1, "Breath analyzer system and method of operating the same", 29-07-2010, Delphi Technologies Inc. It offers a simple IR absorption analyzer with one IR radiation source, one optic cell with gas inlet and outlet, and three detectors with filters. One detector with a filter is intended for ethanol vapor absorption IR band, second detector with a filter is intended for $CO_2$ absorption IR band, and the third one detector with a filter is intended for the water vapor absorption IR band. Spectral band of $CO_2$ referred to as 4.4 $\mu$m is employed considering that the expected $CO_2$ concentration in the exhaled air is rather high. However no other spectral bands of $CO_2$ or different isotopic species of $CO_2$ are considered, and by the known system and method is no way to measure of precision measurement of low $CO_2$ concentrations and to extend measurement range.

Technical problem to be solved

Present invention aims to improve the dynamic detection range of $CO_2$ concentration measurement (up to six orders of magnitude of total measurement range) and to extend measurement range in a simple and compact setup.

Disclosure of invention

The essential solution to the problem according to the proposed invention is achieved by the proposed device Optical $CO_2$ concentration meter based on IR light absorption in gas according to claims 1-2.

Advantages of the invention

The invention provides the dynamic range from 1 ppm to $10^6$ ppm of $CO_2$ gas concentration measurement using optical $CO_2$ concentration meter without loss of measurement linearity, measuring in the same chamber and maintaining the measurement accuracy of $\pm$1% with the optic path length of 2 cm and sample volume of 3 ml.

It is known that many elements in the nature exist as different isotopes. For example, stable isotopes of carbon $^{12}C$ and $^{13}C$ and correspondingly, the isotopic species $^{12}CO_2$ and $^{13}CO_2$, are found in the ratio 89.9 on Earth, the said ratio remaining extremely stable and deviating by only $10^{-4}$ parts in natural conditions and in many industrial setups. Whereas, for the most applications, measurement of $CO_2$ concentration would be considered acceptable at a precision of 5 % or similar. Therefore, at this point, natural ratio of $^{12}CO_2/^{13}CO_2$ abundance can be taken as a reference constant value.

On the other hand, it is known that molecular gases that are composed of different isotope atoms may feature slightly different spectra, in particular, different vibrational - rotation spectra. Many of these bands overlap, but some of them can be distinguished. Such distinguishable bands are for example vibrational-rotational features $v_3$ of $^{12}CO_2$ in the range 4.2 $\mu$m - 4.35 $\mu$m versus the corresponding features $v_3$ of $^{13}CO_2$ in the range 4.35 $\mu$m - 4.45 $\mu$m.

The present invention proposes to detect $CO_2$ concentration by employing $^{12}CO_2$ IR absorption band in the case of low $CO_2$ concentrations and by employing the $^{13}CO_2$ IR absorption band in the case of high $CO_2$ concentrations, within the same optic cell. In practice, the different measurement ranges may not be needed "switched" physically at all. All beams and detectors would work simultaneously and only after collection of data the best detection mode will be decided by the software algorithm and the most accurate results will be calculated. Besides of the two beams mentioned above, the third beam is preferred to be used as a reference one, in the range of IR unabsorbed by any species of interest, to compensate for any drift and fluctuations in the optical system. The reference beam helps to improve signal-to-noise ratio, thus it improves the detection limit and extends the measurement range into the lower concentrations.

Brief description of the drawings

Fig. 1 - A block diagram of the optical $CO_2$ concentration meter based on gas absorption.

Fig. 2 - Theoretical roto-vibrational absorption spectra of the $^{12}CO_2$ molecule in a 4.2-4.45 $\mu$m wavelength range (according to the HITRAN database).

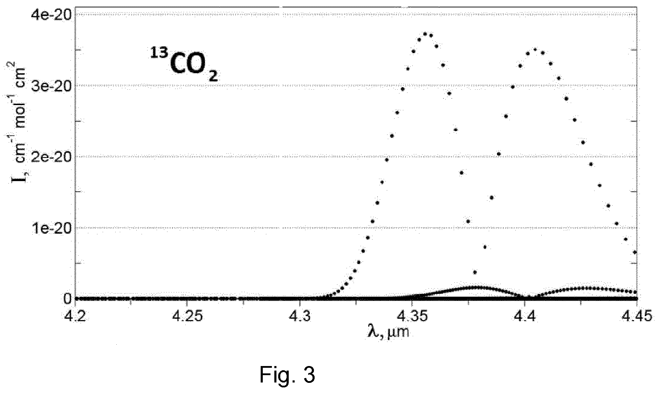

Fig. 3 - Theoretical roto-vibrational absorption spectra of the $^{13}CO_2$ molecule in a 4.35-4.45 $\mu$m wavelength range (according to the HITRAN database).

Fig. 4 - A block diagram of the analyzer.

Fig. 5 - A temporal algorithm of measurement.

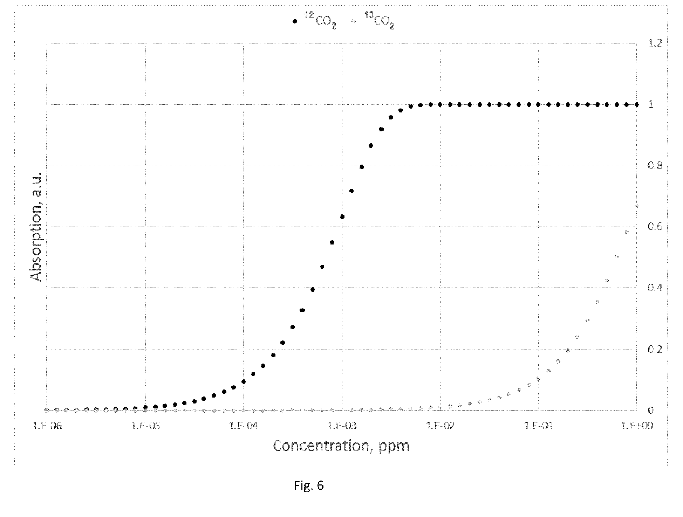

Fig. 6 - Example of the saturation of the measured $^{12}CO_2$ signal due to 100-fold concentration increase and an appearance of the $^{13}CO_2$ signal for the corresponding concentration.

One of the implementation examples of the proposed invention

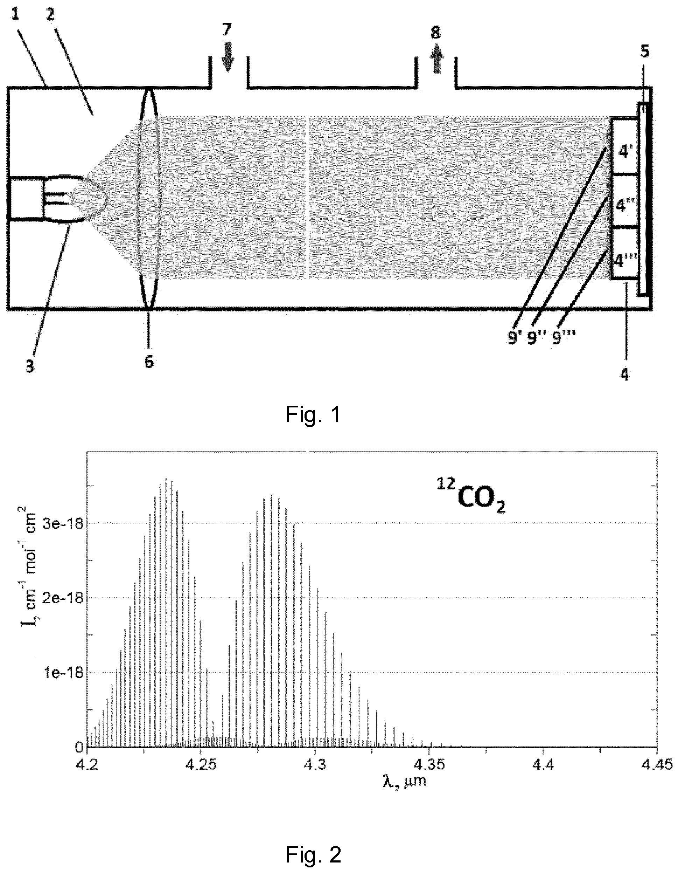

Figure 1 shows a block diagram of the proposed $CO_2$ concentration meter which comprises a casing 1, having a working chamber 2, where at one end of the working chamber 2 is located a light source 3, and at another end, in front of the light source 3, a detector's node 4 is installed, which is consisting of three detectors (4', 4", 4") and an analyzer 5. Between the light source 3 and the detector's node 4, a lens 6 is placed, which directs the light from the source 3 to a surface of the detector (4', 4", 4"). For introducing testing gas an entrance into chamber 2 is made in a casing opening 7 and outlet 8 for gas released from the chamber 2. The first, the second and third detectors (4', 4", 4") are respectively covered with different filters (9', 9", 9‴), which are configured to transmit the passing light at particular wavelength ranges 3.4-4.0 $\mu$m, 4.2-4.35 $\mu$m and 4.35-4.45 $\mu$m respectively. The first detector 4' is dedicated for measuring of light intensity of unabsorbed light at the wavelength of 3.4-4.0 $\mu$m as a reference signal. The second detector 4" is sensitive to $^{12}CO_2$ absorption of roto-vibrational oscillations at wavelength of 4.2-4.35$\mu$m and measures $CO_2$ concentration in the range of 2-500 ppm. The third detector 4‴ is sensitive to $^{13}CO_2$ absorption of roto-vibrational oscillations at wavelength of 4.35-4.45 $\mu$m and measures $CO_2$ concentration in the range of 500-$10^6$ ppm.

500-$10^6$ ppm $CO_2$ concentration is estimated as follows: up to 500 ppm $^{12}CO_2$ is measured by the second (4") detector sensitive to 4.2-4.35 $\mu$m wavelength light by comparing detector's signal with the first signal of the reference detector (4'). When the $CO_2$ concentration increases the third detector (4‴) effectively measures the signal of $^{13}CO_2$ due to the increase of intensity of $^{13}C$ roto-vibrational oscillations in the 4.35-4.45 $\mu$m wavelength range and compares it with the first signal of the reference detector (4'). In this way by comparing the signals of the three detectors, the $CO_2$ concentration is very accurately determined in the range of 1-$10^6$ppm without additional measures (e.g. dilution or sophisticated multicomponent system).
Signal comparison and determination takes place in detector signal analyzer 5, which can also be a spectro-meter, if optical $CO_2$ concentration meter is part of it and not an independent device.

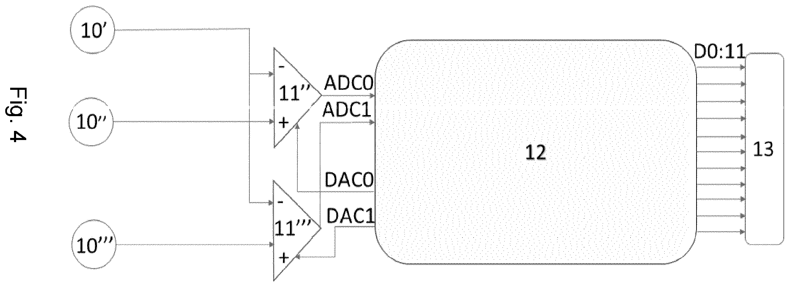

Figure 4 shows a block diagram of the signal analyzer 5 of proposed optical $CO_2$ concentration meter, designed to measure $CO_2$ automatically over a wide concentration range according to $^{12}C$ and $^{13}C$ isotope roto-vibrational oscillations absorption lines while maintaining signal linearity, which transmits its output signals to the data output device 13 (e.g. computer). Differential amplifiers (11", 11‴) are dedicated for amplification and comparison of detector (as in Figure 1: reference 4', and either 4" or 4‴) signals to balance the system during the first measurement and to select automatically the required differential amplifier according to the signal intensity data. In case of measuring low $CO_2$ gas concentration the differential amplifier 11" is used - the registered $^{12}CO_2$ signal (10") and the reference (10') signals are compared. In the case of high $CO_2$ gas concentration (according to $^{13}CO_2$) the differential amplifier 11‴ is used - the registered (10‴) and reference (10') signals are compared. Processes in the digital control unit 12 (e.g. in the CORTEX-M3 processor) are executed according to the temporal algorithm (Figure 5). $CO_2$ gas can be measured in stationary mode or in gas flow mode - in both modes a certain measurement time (e.g. 100s) is selected, during which in unit 12 the number of pulses registered in the detector is summed.
Figure 5 shows an algorithm of the temporal measurement process of the proposed optical $CO_2$ concentration meter, consisting of the following stages:

A - system cleaning,

B - system balancing,

C - measurement procedures (same for calibration gas and testing gas).

In stage A - system purging with N$_2$ gas is carried out ensuring a low CO$_2$ gas background.

In stage B - system balancing is carried out, which is necessary for ensuring the measurements accuracy, which depends on the same initial signal voltage in all three detectors when measuring reference gas (background signal). Therefore, in stage B, the same voltage of the detector signals is ensured. The signals from the differential amplifiers 11" and 11‴ are analyzed in the digital control unit 12 and if the condition of the algorithm is satisfied (output 0 - signals are equal ) - a digital signal/result is issued what measurement can be started, but if the algorithm is not satisfied ( output 1 - signals are not equal) - the correction signal needed to balance the system is issued via Digital-Analog Converter : DAC0 or DAC1 are transmitted depending on which detector is generated the difference. The process is performed iteratively until the condition of the algorithm is satisfied.

In step C, the measurement duration (e.g. 100s) and the sampling frequency - number of discretization cycles N is set (e.g. every 0.1s, N=1000) and photometry of the measured gas is performed until the number of discretization N cycles is achieved. For every discrete measurement $\delta \geq (D)$ condition is checked, wherein $\delta$ is IR light absorption by the spectral band $v_3$ of $^{12}CO_2$ in the sample. When D=0.75 the measured ($^{12}CO_2$) 11" signal saturates due to the (exponential) concentration increase. Accordingly, depending on the $\delta$ value, either data array 11" or 11‴ is filled in, also the value of $\delta$ is stored. All the results are recorded and visualized by the registration and visualization device (13) (e.g. computer) at the end of the measurement, then the measurement algorithm is stopped. The light source (3) can be any broadband (incoherent) light source: incandescent lamp, LED - lamp (wavelength 3.8-4.6 $\mu$m), etc. If using a 3.8-4.6 $\mu$m LED lamp, the lens (6) shown in Fig.1 function is performed by an integrated forward-focusing special light filter, therefore, no light-focusing lens or reflective mirrors are needed. The first, second and third detectors can be uncooled photodiodes. They can also be photodiodes with the preamplifiers (e.g. LMS43PD-05-CG-RPA or similar).

Selective filters are required for the device: reference filter of preferred wavelength 3.4-4.0$\mu$m (bandwidth centered at 3.7$\mu$m), $^{12}CO_2$ sensitive filter at the preferred 4.2-4.35 $\mu$m wavelength range (bandwidth centered at 4.28 $\mu$m), and $^{13}CO_2$ sensitive filter at the preferred 4.35-4.45 $\mu$m wavelength range (bandwidth centered at 4.4$\mu$m). Individually designed filters of resolutions of 100nm or better should be used for the instrument.

In examples not falling under the scope of the invention, there is also the possibility to measure CO gas after selecting the appropriate wavelength filters (9' - 9‴) for the detectors (4' - 4‴): $^{12}CO$ sensitive filter at the 4.55-4.8 $\mu$m wavelength range and $^{13}CO$ sensitive filter at the 4.65-4.9 $\mu$m wavelength range by using the same light source (3), camera (1), detectors unit (4) and signal analyzer (5).

## Claims

1. An optical CO$_2$ concentration meter based on IR light absorption, comprising a casing (1), having a working chamber (2), wherein at an one end of the working chamber (2) a light source (3) is located and at another end, in front of the light source (3), a detector unit (4) is installed, which includes three detectors (4', 4", 4"), each of the detectors is provided respectively with a different narrow-band IR filter (9', 9", 9‴) and an analyzer (5), wherein a lens (6) is located between the light source (3) and the detector unit (4), which is configured to direct the light to surfaces of the detectors (4', 4", 4") through filters (9', 9", 9‴), the casing (1) has at least one inlet (7) for introducing the CO$_2$ gas into the chamber (2) and at least one outlet (8) designed to release gas from the chamber (2), wherein

   the filter (9') is configured to transmit the IR light unabsorbed by any of CO$_2$ isotopic species, preferably in a range 3.4 - 4.0 $\mu$m, the second filter (9") is configured to transmit an IR spectral band $v_3$ absorbed solely by isotopic species $^{12}CO_2$, preferably in a range 4.2 - 4.35 $\mu$m, and the third filter (9‴) is configured to transmit an IR spectral band $v_3$ absorbed solely by isotopic species $^{13}CO_2$, preferably in a range 4.35 - 4.45 $\mu$m,, wherein all detectors (4', 4", 4") via the analyzer (5) and differential amplifiers 11" and 11 "' are connected to a digital control unit (12), wherein a digital control unit (12) is configured to execute a temporal algorithm to purge the optic chamber with clean gas N$_2$, to balance differential amplifiers' 11" and 11‴ voltages and to fill the working chamber 2 with the measured gas, **characterized in that**
   the control unit (12) is configured to perform additional steps of the temporal algorithm in order:

   - to record a differential signal from the differential amplifier (11") obtained by comparing signals of the detector (4") versus the detector (4') for low CO$_2$ concentration measurement range and to check as to whether or not the condition of a signal saturation is achieved, wherein the condition of the signal saturation being the IR light absorption by the spectral band $v_3$ of $^{12}CO_2$ in the sample, $\delta$, to appear equal or greater than a predetermined threshold value D, and
   - in case that the signal saturation is not achieved then to record a data array from the differential amplifier (11") for low concentration measurement range,
   - in case that the signal saturation is achieved to record a differential signal from the differential amplifier (11‴)

obtained by comparing signals from the detector (4‴) versus the detector (4') for high concentration measurement range.

2. A system for metering of $CO_2$ concentration, **characterized in that** it comprises the meter according to claim 1.

**Patentansprüche**

1. Optischer $CO_2$-Konzentrationsmesser auf der Basis von IR-Lichtabsorption, umfassend ein Gehäuse (1) mit einer Arbeitskammer (2), wobei sich an einem Ende der Arbeitskammer (2) eine Lichtquelle (3) befindet und an einem anderen Ende vor der Lichtquelle (3) eine Detektoreinheit (4) installiert ist, die drei Detektoren (4', 4", 4‴) beinhaltet, wobei jeder der Detektoren jeweils mit einem unterschiedlichen Schmalband-IR-Filter (9', 9", 9‴) und einem Analysator (5) bereitgestellt ist, wobei sich eine Linse (6) zwischen der Lichtquelle (3) und der Detektoreinheit (4) befindet, die dazu konfiguriert ist, das Licht auf Oberflächen der Detektoren (4', 4", 4‴) durch Filter (9', 9", 9‴) zu richten, wobei das Gehäuse (1) zumindest einen Einlass (7) zum Einleiten des $CO_2$-Gases in die Kammer (2) und zumindest einen Auslass (8) aufweist, der dazu ausgelegt ist, Gas aus der Kammer (2) freizusetzen, wobei

der Filter (9') dazu konfiguriert ist, dass IR-Licht durchzulassen, das durch eine beliebige von $CO_2$-Isotopenspezies nicht absorbiert wird, bevorzugt in einer Spanne von 3,4-4,0 $\mu$m, der zweite Filter (9") dazu konfiguriert ist, ein IR-Spektralband $v_3$ durchzulassen, das ausschließlich durch Isotopenspezies $^{12}CO_2$ absorbiert wird, bevorzugt in einer Spanne von 4,2-4,35 $\mu$m, und der dritte Filter (9‴) dazu konfiguriert ist, ein IR-Spektralband $v_3$ durchzulassen, das ausschließlich durch Isotopenspezies $^{13}CO_2$ absorbiert wird, bevorzugt in einer Spanne von 4,35-4,45 $\mu$m, wobei alle Detektoren (4', 4", 4‴) über den Analysator (5) und die Differenzverstärker 11" und 11‴ mit einer digitalen Steuereinheit (12) verbunden sind, wobei eine digitale Steuereinheit (12) dazu konfiguriert ist, einen zeitlichen Algorithmus auszuführen, um die optische Kammer mit Reingas $N_2$ zu spülen, um Spannungen der Differenzverstärker 11" und 11‴ auszugleichen und um die Arbeitskammer 2 mit dem gemessenen Gas zu füllen, **dadurch gekennzeichnet, dass**
die Steuereinheit (12) dazu konfiguriert ist, zusätzliche Schritte des zeitlichen Algorithmus in Reihenfolge durchzuführen:

- um ein Differenzsignal von dem Differenzverstärker (11") aufzuzeichnen, das durch Vergleichen von Signalen des Detektors (4") versus dem Detektor (4') auf niedrige $CO_2$-Konzentrationsmesspanne erhalten wird und um zu prüfen, ob der Zustand einer Signalsättigung erreicht ist oder nicht, wobei der Zustand der Signalsättigung ist, dass die IR-Lichtabsorption durch das Spektralband $v_3$ von $^{12}CO_2$ in der Probe, $\delta$, gleich einem oder größer als ein vorbestimmter Schwellenwert D erscheint,
und
- falls die Signalsättigung nicht erreicht ist, dann ein Datenarray aus dem Differenzverstärker (11") für niedrige Konzentrationsmesspanne aufzuzeichnen,
- falls die Signalsättigung erreicht ist, ein Differenzsignal von dem Differenzverstärker (11‴) aufzuzeichnen, das durch Vergleichen von Signalen von dem Detektor (4‴) versus dem Detektor (4') für hohe Konzentrationsmessspanne erhalten wird.

2. System zum Messen von $CO_2$-Konzentration, **dadurch gekennzeichnet, dass** es den Messer nach Anspruch 1 umfasst.

**Revendications**

1. Compteur optique de concentration de $CO_2$ basé sur l'absorption de lumière IR, comprenant un boîtier (1), ayant une chambre de travail (2), dans lequel à une extrémité de la chambre de travail (2) se trouve une source de lumière (3) et à une autre extrémité, face à la source de lumière (3), est installée une unité de détection (4), qui comprend trois détecteurs (4', 4", 4‴), chacun des détecteurs est muni respectivement d'un filtre IR à bande étroite différent (9', 9", 9‴) et d'un analyseur (5), dans lequel une lentille (6) est située entre la source de lumière (3) et l'unité de détection (4), laquelle est configurée pour diriger la lumière vers les surfaces des détecteurs (4', 4", 4‴) à travers des filtres (9', 9", 9‴), le boîtier (1) a au moins une entrée (7) permettant l'introduction du gaz $CO_2$ dans la chambre (2) et au moins une sortie (8) conçue pour évacuer le gaz de la chambre (2), dans lequel

le filtre (9') est configuré pour transmettre la lumière IR non absorbée par l'une quelconque des espèces

isotopiques $CO_2$, de préférence dans une plage de 3,4 à 4,0 $\mu$m, le deuxième filtre (9") est configuré pour transmettre une bande spectrale IR $v_3$ absorbée uniquement par les espèces isotopiques $^{12}CO_2$, de préférence dans une plage de 4,2 à 4,35 $\mu$m, et le troisième filtre (9''') est configuré pour transmettre une bande spectrale IR $v_3$ absorbée uniquement par les espèces isotopiques $^{13}CO_2$, de préférence dans une plage de 4,35 à 4,45 $\mu$m, dans lequel tous les détecteurs (4', 4", 4''') via l'analyseur (5) et les amplificateurs différentiels 11" et 11''' sont connectés à une unité de commande numérique (12), dans lequel une unité de commande numérique (12) est configurée pour exécuter un algorithme temporel pour purger la chambre optique avec du gaz propre $N_2$, pour équilibrer les tensions des amplificateurs différentiels 11" et 11''' et pour remplir la chambre de travail 2 avec le gaz mesuré, **caractérisé en ce que**

l'unité de commande (12) est configurée pour effectuer des étapes supplémentaires de l'algorithme temporel afin de :

- enregistrer un signal différentiel provenant de l'amplificateur différentiel (11") obtenu en comparant les signaux du détecteur (4") à ceux du détecteur (4') pour une plage de mesure à faible concentration de $CO_2$ et vérifier si la condition de saturation de signal est ou n'est pas atteinte, dans lequel la condition de saturation de signal étant l'absorption de lumière IR par la bande spectrale $v_3$ de $^{12}CO_2$ dans l'échantillon, $\delta$, pour apparaître égale ou supérieure à une valeur seuil prédéterminée D, et

- dans le cas où la saturation de signal n'est pas atteinte, alors enregistrer une matrice de données provenant de l'amplificateur différentiel (11") pour une plage de mesure de faible concentration,

- dans le cas où la saturation de signal est atteinte, enregistrer un signal différentiel provenant de l'amplificateur différentiel (11''') obtenu en comparant les signaux provenant du détecteur (4''') à ceux du détecteur (4') pour une plage de mesure de concentration élevée.

2. Système de mesure de concentration de $CO_2$, **caractérisé en ce qu'**il comprend le compteur selon la revendication 1.

Fig. 1

Fig. 2

Fig. 3

Fig. 4

Fig. 5

Fig. 6

**EP 4 276 444 B1**

**Patent documents cited in the description**

- LT 2022519
- US 5747809 A
- US 2003205673 A1
- KR 101835556 B1
- US 2010188232 A1